# EUROPEAN PATENT SPECIFICATION

(11) **EP 3 672 512 B1**
(45) Date of publication and mention of the grant of the patent: **16.07.2025**
(21) Application number: 18848165.9
(22) Date of filing: 27.08.2018
(51) Int. Cl.: A61B 18/14, A61M 25/00, A61B 34/00, A61B 17/32, A61B 17/04, A61B 17/221, A61B 17/3205, A61B 17/00, A61B 18/00, A61B 34/30, A61B 90/00, A61M 25/01, A61M 25/06, A61F 2/24, A61B 17/22

(54) **CATHETERS AND MANIPULATORS WITH ARTICULABLE ENDS**
KATHETER UND MANIPULATOREN MIT GELENKIGEN ENDEN
CATHÉTERS ET MANIPULATEURS À EXTRÉMITÉS ARTICULÉES

(30) Priority: 25.08.2017 US 201762550347 P; 02.10.2017 US 201762567203 P; 27.04.2018 US 201862663518 P; 21.06.2018 US 201862688378 P; 30.07.2018 US 201862712194 P
(43) Date of publication of application: 01.07.2020
(73) Proprietor: Transmural Systems LLC, Andover, MA 01810 (US); The United States of America, as represented by the Secretary, Department of Health and Human Services, Bethesda, MD 20852-7660 (US)
(72) Inventor: LEDERMAN, Robert J., Chevy Chase, Maryland 20815 (US); KHAN, Jaffar, Bethesda, Maryland 20814 (US); ROGERS, Toby, Bethesda, Maryland 20814 (US); RAFIEE, Nasser, Andover, MA 01810 (US); MACDONALD, Stuart, Andover, MA 01810 (US); HOUSE, Morgan, Andover, MA 01810 (US)
(74) Representative: Hancox, Jonathan Christopher
(86) International application number: PCT/US2018/048177
(87) International publication number: WO 2019/040943

(56) References cited:
- EP-A1- 3 175 813
- WO-A1-2018/009718
- DE-A1- 10 037 660
- DE-A1- 10 037 660
- DE-U1- 202010 016 945
- DE-U1- 202010 016 945
- US-A- 4 493 320
- US-A1- 2008 015 409
- US-A1- 2016 317 174
- US-B1- 9 282 993

## Description

### CROSS-REFERENCE TO RELATED APPLICATION

This patent application is related to U.S. Provisional Application Serial No. 62/550,347, filed August 25, 2017, U.S. Provisional Application Serial No. 62/567,203, filed October 2, 2017, U.S. Provisional Patent Application Serial No. 62/663,518, filed April 27, 2018, U.S. Provisional Application Serial No. 62/688,378, filed June 21, 2018, and U.S. Provisional Patent Application Serial No. 62/712,194, filed July 30, 2018.

### PRIOR ART

WO2018/009718 (Revive Medical LLC) relates to methods and devices for valve clip excision.

### FIELD

The invention relates to an elongate catheter in accordance with claim 1, a system in accordance with claim 12 and an electrosurgical system in accordance with claim 13.

### BRIEF DESCRIPTION OF THE FIGURES

Figs. 1A-1D are illustrations of a first device in accordance with the present disclosure.
Figs. 2A-2D are illustrations of a second device in accordance with the present disclosure.
Figs. 3A-3C show aspects of a first embodiment of a guide wire used in an electrosurgical procedure in accordance with the disclosure.
Fig. 4 shows aspects of a second embodiment of a guide wire used in an electrosurgical procedure in accordance with the disclosure.
Figs. 5A-5B present views of a grasping catheter or manipulator in accordance with the present disclosure.
Fig. 5C presents a further embodiment of a grasping catheter in accordance with the present disclosure.
Fig. 6 illustrates a cross sectional view of an extruded main body portion of an illustrative catheter in accordance with the disclosure.
Figs. 7A-7D present various embodiments of a dual lumen catheter in accordance with the present disclosure.
Fig. 8 presents the embodiment of Fig. 7A including a snare catheter disposed through the minor lumen for effectuating capture, for example, of a guidewire in a medical procedure.
Figs. 9A-9B illustrate an articulating catheter having two preformed bends that resume their bent shape when advanced distally from the main catheter.
Fig. 10 illustrates an illustrative cross section of a catheter in accordance with the present disclosure.
Figs. 11A-11C present various views of a further embodiment of a catheter in accordance with the present disclosure.
Figs. 12A-12E present views of still a further catheter in accordance with the present disclosure.
Figs. 13A-13C present views of a procedure using the embodiment of Figs. 12A-12E with respect to the anatomical structure of a tricuspid valve.

### DETAILED DESCRIPTION

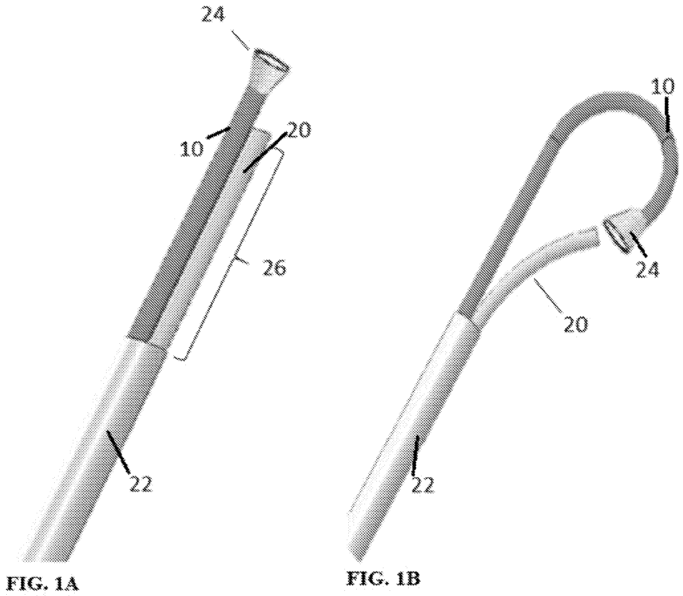

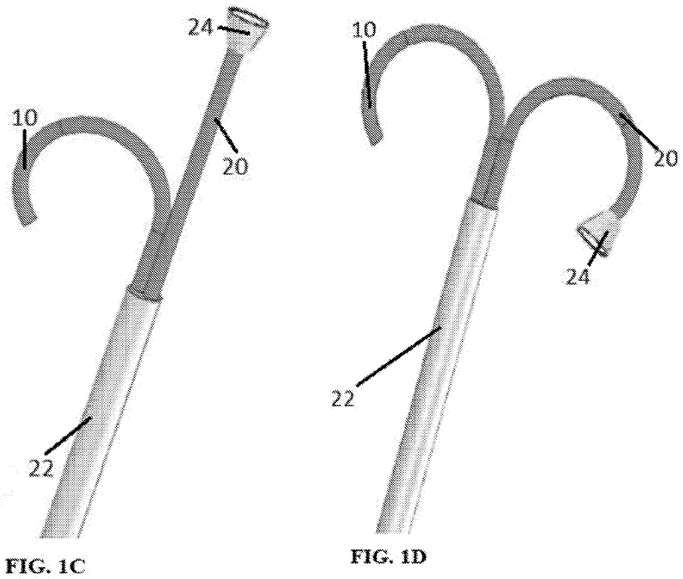

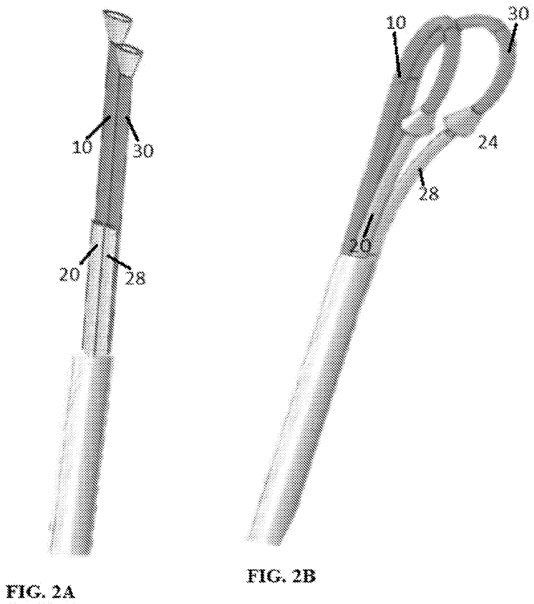

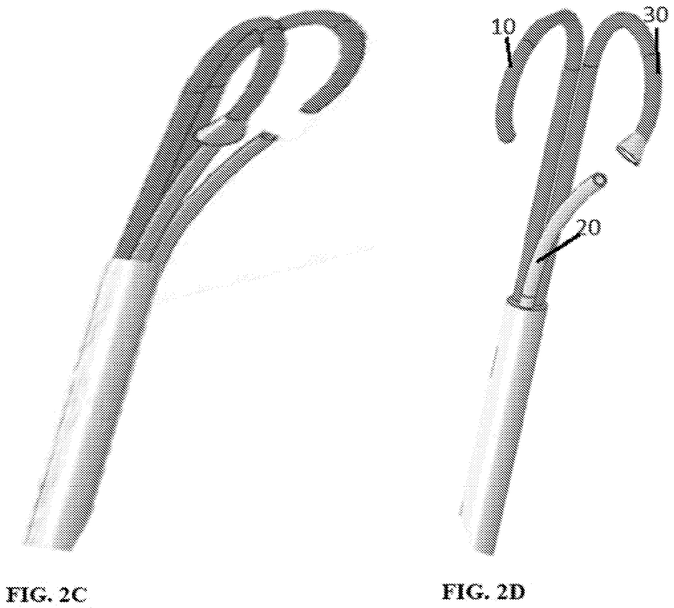

For purposes of illustration, and not limitation, exemplary embodiments of a catheter, which can also be used as a robotic manipulator, are presented in Figs. 1A-1D and 2A-2D. For purposes of simplicity but not limitation, the devices are typically referred to herein as "catheters" but it will be understood by those of skill in the art that they can equally be considered to be robotic manipulators.

With reference to Figs. 1A-1D, an elongate catheter is provided having a proximal end and a distal end. The catheter includes an elongate tubular main body 22 having a proximal end, a distal end, and defining at least one elongate passage therethrough. The elongate tubular main body defining a longitudinal axis along its length.

The catheter includes a first elongate inner body 10 having a proximal end and a distal end. The inner body 10 is illustrated with an illustrative cone-shaped atraumatic distal tip 24 that is configured to spread applied stress out over a larger area, which can be of particular benefit when contacting delicate anatomical structures. The first elongate inner body 10 is slidably disposed within the at least one elongate passage of the elongate tubular main body 22.

Also illustrated is a second elongate inner body 20 having a proximal end and a distal end that is slidably disposed within the at least one elongate passage of the elongate tubular main body 22, which is suitably configured to maintain registration of bodies 10 and 20 with respect to each other and hold them together. Bodies 10 and 20 can be housed in a common passage, or in individual passages defined within body 22. Body 20 is slidably disposed with respect to the first inner body 10, wherein an exposed distal region 26 of body 20 is illustrated as protruding beyond the distal end of main body 22.

As illustrated, the distal end of the first and second inner elongate bodies 10, 20 are preferably biased or otherwise configured to be curled or steered away from the longitudinal axis in a proximal direction when the first elongate inner body is advanced distally with respect to the main body by virtue of inner body 10 being removed from body 22. Bodies 10, 20 can be configured to curl as illustrated when advanced distally from body 22 by making bodies 10, 20 at least in part from shape memory materials, and/or by utilizing a steering wire that travels the length of the body 10, 20 that is attached to a distal end of each of the bodies 10, 20, such as by way of a ring (e.g., a radiopaque marker band) that is attached to the distal end of the bodies. In another embodiment, one or more of bodies 10, 20 can be formed at least in part by thermoplastic or other polymeric or composite material that is molded with a preformed bend therein. Such a pre-bent or pre-formed body 10, 20 can then be loaded, for example, into main body 22, wherein main body 22 maintains the bodies 10, 20 in a straight orientation until they bodies 10, 20 are advanced distally with respect to main body 22, at which time they revert to their curved shape and regain at least some of their original curvature.

Main body 22 can simply be an overwrap or a sheath in some implementations that functions to maintain the bodies 10, 20 in a parallel relationship and optionally maintains the bodies 10, 20 in a relative orientation until the bodies 10, 20 are advanced distally with respect to body 22. In other implementations, body 22 can be more sophisticated such as a multi-lumen extrusion including a plurality of lumens for slidably containing bodies 10, 20, and other devices, as desired. In lieu of a main body 22 or overwrap, bodies 10, 20 can alternatively be fused or adhered to each other, or be provided with an adjustable coupling that runs their lengths that permits relative slidability of bodies 10, 20. For example, body 10 can be provided with a rib along the majority of its length (e.g., except for the distal most 5-10cm) having a "T"-shaped cross section, wherein the base of the T adjoins the body 10, and body 20 can be provided with a "C"-shaped channel along its length that slidably receives the T-shaped rib.

If desired, each of the first elongate inner body 10 and second elongate inner body 20 can each define one or more lumens along their respective lengths. The lumen(s) can be used, for example, for passage of a further medical instrument such as a guidewire or viewing scope, for directing electrical conductors, and the like, and/or for passage of a steering wire along the length of body 10, 20 terminating, for example, in a marker band at the distal end of body 10, 20 that the steering wire attaches to. Other examples of suitable steering mechanisms can be found in U.S. Patent No. 6,030,360, and U.S. Patent No. 6,579,278. Either body 10, 20 if equipped with such a passage can additionally or alternatively include a movable body (e.g., core wire, snare catheter, etc.) slidably disposed therein.

If the passage within body 10 includes a snare catheter (such as that described in U.S. Patent Application Serial No. 13/824,198, filed May 1, 2013, which is annexed to U.S. Provisional Application Serial No. 62/567,203, filed October 2, 2017, as US2013/0211510), the snare catheter can be directed out of the distal end of body 10 to provide a landing or target zone for a guidewire that is directed through the distal end of body 20 (not shown). This permits a guidewire that traverses through the distal end of the body 20 to be captured by the snare catheter that extends outwardly from body 10, thereby permitting the guidewire extending from the distal end of body 20 to be pulled into the distal end of body 10, and advanced through the body 10 and externalized or otherwise directed out of the proximal end of body 10 (not shown).

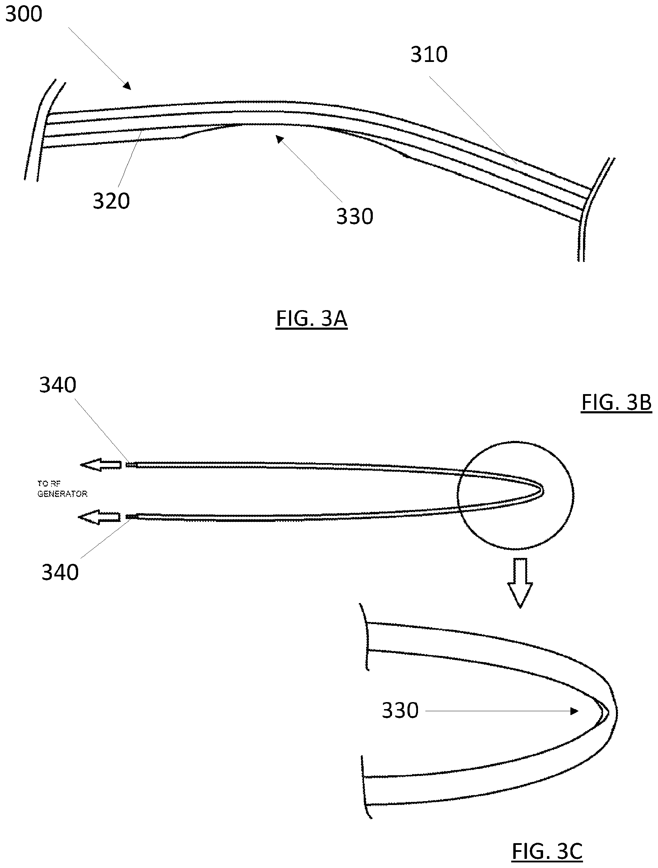

If desired, the guidewire disposed in body 20 can include an electrically conductive core wire surrounded by a jacket made from dielectric/insulating material. The jacket can be removed from a portion of the core wire to expose a portion of the core wire. In a further embodiment, as illustrated in Figs. 3A-3C, the guidewire can include a core wire that is in turn surrounded by a first insulating layer. As illustrated in Fig. 3A, the guidewire 300 can have an electrically conductive core wire 320 surrounded by a jacket 320 made from dielectric material, such as PTFE or other suitable material. The jacket can be stripped off on one side to create an exposed region 330 of the core wire 320. The ends of the core wire 320 can likewise be exposed, and the wire can be bent in half so that the exposed core wire 320 faces itself. When the exposed ends 340 of the core wire 320 are then connected to a generator (not shown) in a bipolar arrangement in this case to cause current to pass through the core wire, in the exposed region of the core wire that is bent over, an electrical discharge, or arc, can develop that jumps across the gap (rather than the current passing only along the core wire) that can be used to help cut and/or burn through tissue by pulling the exposed wire through the tissue.

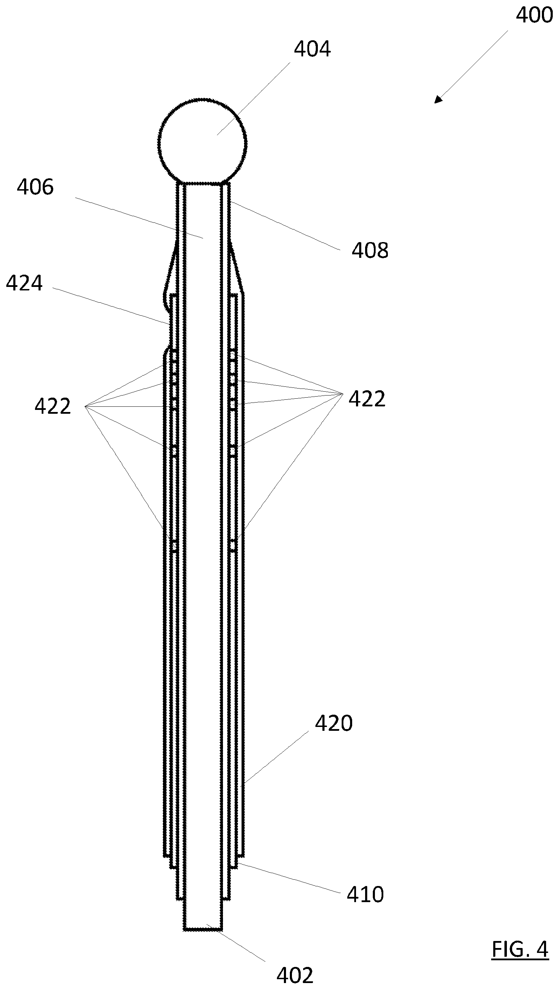

If desired, the guidewire can be provided with more than one conducting layer as embodiment 400 in Fig. 4. Guidewire 400 has an exposed proximal end 402 connected to a distal tip (in this case in the shape of a metallic ball 404, and an elongate core wire 406. A first insulating layer 408 (made of a polymeric layer, for example), is disposed about the core wire 406 along its length, but leaving the tip 404 and proximal end 402 exposed. Proximal end 402 can be electrically coupled to a signal generator, and the current can pass, for example, through the distal tip 404 and follow a return path to a conductive path (not shown) through the patient's body (monopolar arrangement) to the electrical generator. This is a useful arrangement for cutting through tissue with the tip of the guidewire 400. Guidewire 400 further includes a second electrical conductor, or conducting layer, 410, is disposed at least partially about, or at least radially outwardly from, the first insulating layer 408. The second electrical conductor/conducting layer 412, in turn, can in turn be surrounded by an outer insulating layer 420. The outer insulating layer can be removed to expose a portion of the second electrical conductor/conducting layer to define an exposed portion 424 of the second electrical conductor/conducting layer. As illustrated, portion 424 is facing laterally outwardly to permit a cut to be performed by moving the guidewire 400 laterally to the side, when a proximal end of the layer 410 is attached to a signal generator. Current then flows through the exposed portion 424 and through the tissue to a conductive pad that is attached to a return path of the signal generator. Conductive layer 410 can be a continuous layer, such as a tubular layer, or can be an interrupted layer, wherein a conductive path is nonetheless maintained from the exposed patch 424 to the proximal end of the conductive layer 410.

Conductive layer 410 can be formed, for example, from a metallic tube, such as a hypotube, in turn be defined by a tubular body that defines at least one opening 422 therethrough. For example, the at least one opening can be spiral shaped (via laser cutting) and winds around the first insulating layer, resulting in the remaining conductive material also winding around the first insulating layer. Alternatively, the at least one opening and the tubular body define a plurality of articulating segments, similar to those defined in U.S. Patent No. 8,530,783, February 3, 2010, U.S. Patent No. 5,605,543, filed January 30, 1996, U.S. Patent Application Serial No. 10/969,088, filed October 20, 2004, or WO2017117092, and appended to U.S. Provisional Application Serial No. 62/567,203, filed October 2, 2017.

The disclosure also provides an electrosurgical system including a radio frequency power supply (such as that described in U.S. Patent No. 6,296,636, which is annexed to U.S. Provisional Application Serial No. 62/567,203, filed October 2, 2017) operably coupled to the electrically conductive core wire of the elongate catheters (and/or of the second conductors of catheters) disclosed herein. Thus, the radio frequency power supply can be operably (and selectively) coupled to the electrically conductive core wire and to the second electrical conductor, as desired. Similarly, the disclosure also provides an ultrasonic surgical system, such as an ultrasonic scalpel, including an ultrasonic power source, such as that disclosed in U.S. Patent No. 6,514,267.

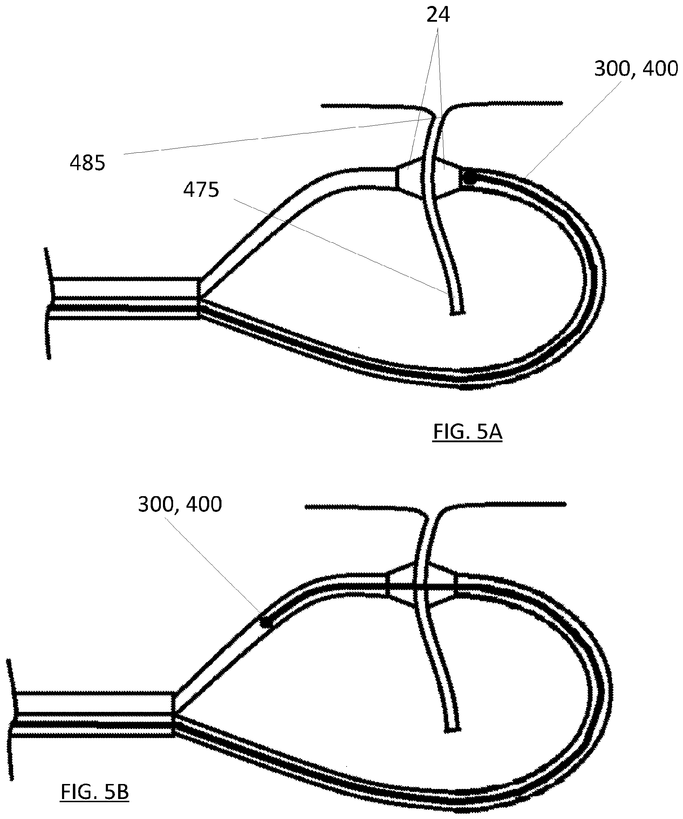

In further embodiments, and with reference to Figs. 5A-5B, the body (e.g., 20) of the catheter can be configured so as to penetrate an anatomical structure, such as a heart valve leaflet 475, prior to passing into the lumen of the first elongate inner body. Tip(s) 24 of the catheter can grip the leaflet and align the passages in the arms of the catheter to permit a guidewire (e.g., 300, 400) to pierce the leaflet and pass through the catheter arms. Piercing can be accomplished (preferably under imaging, such as fluoroscopy) with a sharpened tip and cuff connection, electrosurgical or ultrasonic cutting tip (e.g., 404). Typically, the leaflet (e.g., 475) is penetrated or pierced in a region that is near or in the annulus 485 of the valve leaflet, most preferably where the annulus transitions to the leaflet base. The disclosed embodiments can be used to perform the procedures described in the journal publications annexed to U.S. Provisional Application Serial No. 62/567,203, filed October 2, 2017 (Khan 2016, Babaliaros 2017). When an electrically exposed portion of the guide wire is in alignment with the leaflet, the ends of the catheter can be withdrawn partially, the electrical current can be turned on, and the exposed portion of the guidewire can be pulled through the leaflet, cutting the leaflet.

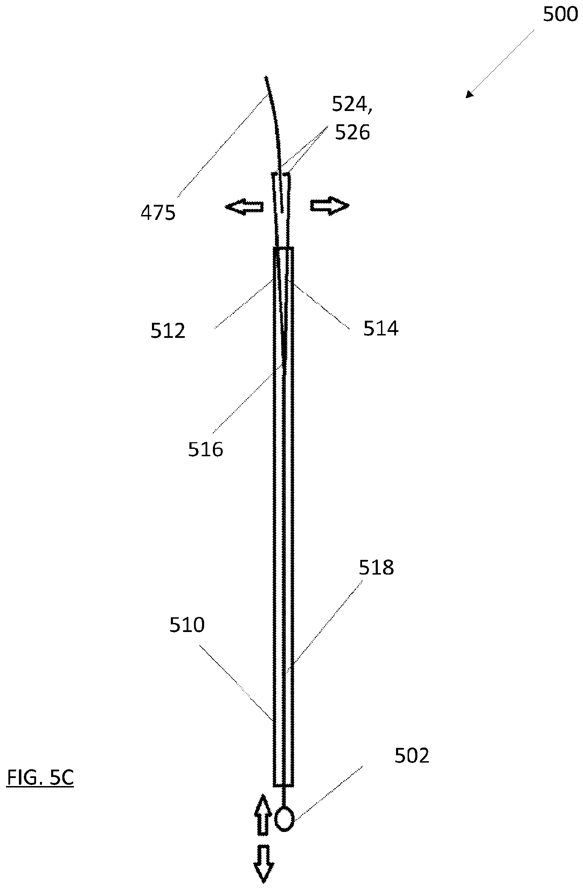

Fig. 5C presents an alternative embodiment of a grasping catheter 500 that can be used in place of a pair of catheters simply for grasping the edge of a leaflet 475. The catheter 500 includes a tubular outer body 510 having a proximal end, a distal end and a longitudinal passage therethrough. An internal slidable gripping mechanism is slidably disposed within the lumen of outer body 510 that includes a proximal actuator or handle 502 that is connected to an elongate inner body 518 that separates at a bifurcation 516 into a first arm 512 and a second arm 514, that in turn terminate in inwardly pointed gripping ends 524, 526. Arms 512, 514 are biased away from each other, and can be urged together by withdrawing the arms and accompanying tips toward the distal end of the tubular member 510. Accordingly, by controlling the relative placement of the inner mechanism and outer tube, the jaws formed by arms 512, 514 and gripping ends 524, 526 can be opened and closed. Catheter 500 can be used as a sub-catheter in any embodiment herein.

As a further example, the movable body (e.g., 20, or a slidable device within a lumen defined by body 20) can include a dart passer that is configured to advance a dart having a suture attached thereto out of the distal end of the second elongate inner body and into a receiving cuff disposed in the lumen of the first elongate inner body, in accordance with the teachings of US2013/0310853. For example, the receiving cuff can be disposed within a lumen defined in body 10 at is attached to a filament/suture that passes through the lumen of body 10 that can receive a dart attached to or resting on the distal end of a hypotube that is advanced through body 20, wherein the dart has a trailing suture that passes through the body of the hypotube. After connecting the dart and cuff, the suture attached to the cuff or the suture attached to the dart can be advanced withdrawing the coupling from the patient, and leaving behind the looped suture.

In accordance with further aspects, the rotational position of the first elongate inner body 10 can be fixed with respect to the rotational position of the second elongate inner body 20. Or, if desired, the rotational positions of each of body 10 and 20 can be controlled by a user at a control actuator/Luer lock at a proximal location of the catheter.

In accordance with further aspects, and as presented in Figs. 2A-2D, the catheter can further include a third elongate inner body 30 having a proximal end and a distal end that is slidably disposed within the at least one elongate passage of the elongate tubular main body, the distal end of the third inner elongate body being biased (or otherwise configured, e.g. via steering wire) to curl away from the longitudinal axis in a proximal direction when the third elongate inner body is advanced distally with respect to the main body. If desired, the catheter can further include a fourth elongate inner body 28 having a proximal end and a distal end that is slidably disposed within the at least one elongate passage of the elongate tubular main body, and slidably disposed with respect to the third inner body. The distal end of the fourth inner elongate body 28 can be biased or otherwise configured to curl away from the longitudinal axis toward the proximally oriented distal end of the third elongate inner body 30 when the fourth elongate inner body is advanced distally with respect to the main body. Any suitable number of such inner bodies can be provided, depending on the procedure being performed.

As with the embodiment of Figs. 1A-1D, the third elongate inner body 30 and fourth elongate inner body 28 can define a lumen along their lengths. The lumen of the fourth elongate inner body 28 can include a device as described elsewhere herein (guidewire, snare catheter) slidably disposed therein having a distal end that is configured to be received by the lumen of the third elongate inner body at the proximally facing distal end of the third elongate inner body.

Each of bodies 10, 20, 22, 28, 30 can be made from a variety of materials, including multilayer polymeric extrusions, such as those described in U.S. Pat. No. 6,464,683 to Samuelson or U.S. Pat. No. 5,538,510 to Fontirroche. Other structures are also possible, including single or multilayer tubes reinforced by braiding, such as metallic braiding material. Any of the catheters, manipulators, guidewires, or other catheters disclosed herein or portions thereof (e.g., portions 10, 20, 22, 28, 30) can be provided with regions of varying or stepped-down stiffness with length using any of the techniques set forth in U.S. Patent No. 7,785,318.

Preferably, the bodies 10, 20, 28, 30 have a decreased stiffness along their length, particularly in their distal regions by adjusting the cross sectional dimensions of the material to impact stiffness and flexibility, while maintaining pushability, as well as the durometer of the material. Hardness/stiffness is described herein with reference to Shore hardness durometer ("D") values. Shore hardness is measured with an apparatus known as a Durometer and consequently is also known as "Durometer hardness". The hardness value is determined by the penetration of the Durometer indenter foot into the sample. The ASTM test method designation is ASTM D2240 00, an example of which is annexed to U.S. Provisional Application Serial No. 62/567,203, filed October 2, 2017. For example, a more proximal region of the catheter can have a durometer of about 72D, an intermediate portion of the catheter (the proximal most 20-30cm of the last 35cm, for example that typically traverses an aortic arch) can have a durometer of about 55D, and the distal 5-10cm of the catheter can have a durometer of about 35D.

Any surface of various components of the system described herein or portions thereof can be provided with one or more suitable lubricious coatings to facilitate procedures by reduction of frictional forces. Such coatings can include, for example, hydrophobic materials such as PolyTetraFluoroEthylene ("PTFE") or silicone oil, or hydrophilic coatings such as Polyvinyl Pyrrolidone ("PVP"). Other coatings are also possible, including, echogenic materials, radiopaque materials and hydrogels, for example.

One or more actuators can be provided to actuate relative proximal and distal movement of bodies 10, 20, 28, 30 with respect to main body 22. Such actuators typically provide either two handles for push-pull actuation, or the actuator can be more exotic. For example, it is also possible to use other actuators as are known in the art, such as threaded rotating actuators similar to those for retractable sheaths as described in U.S. Pat. No. 6,488,694 to Lau and U.S. Pat. No. 5,906,619 to Olson.

With reference to Figs. 3 and 4, the disclosure also provides a method that includes providing an electrosurgical system as described hereinabove, deploying the distal end of the catheter into a patient's vasculature to a target location proximate the patient's valve, deploying the first elongate inner body so that the distal end of the first elongate inner body curls around the edge of the patient's valve leaflet, deploying the second elongate inner body so that the distal end of the second elongate inner body bends toward the distal end of the first elongate inner body, directing the guidewire out of the distal end of the second elongate inner body, through the patient's valve leaflet near the valve annulus (such as where the annulus transitions to the base of the leaflet(, and into the lumen of the first elongate inner body, advancing the guidewire until the exposed portion of the core wire or second conductor is located in a gap defined between the distal end of the first elongate inner body and the distal end of the second elongate inner body that coincides with the valve leaflet, wherein the exposed portion of the core wire is facing in a proximal direction, energizing the power supply of the electrosurgical system, and advancing the exposed portion of the core wire or second conductor through at least a portion of the valve leaflet to effectuate a cut in the valve leaflet.

As described herein, when practicing the illustrative methods, the exposed portion of the core wire or second conductor can be advanced through the valve leaflet through a peripheral edge of the valve leaflet. In some implementations, the valve leaflet can be a mitral valve leaflet, such as a native or artificial/replacement anterior or posterior mitral valve leaflet, or a native or artificial/replacement tricuspid, pulmonary or aortic valve leaflet. It will be appreciated that the disclosed systems can be used with respect to any suitable native or artificial/replacement valve leaflet.

The disclosure also provides a robotic manipulator having a proximal end and a distal end that includes an elongate tubular arm having a proximal end, a distal end, and defining at least one elongate passage therethrough, the elongate tubular arm defining a longitudinal axis along its length. The manipulator further includes a first elongate inner body having a proximal end and a distal end that is slidably disposed within the at least one elongate passage of the elongate tubular arm, the distal end of the first inner elongate body being biased (or otherwise configured, such as pre-forming and/or steering wire) to curl away from the longitudinal axis in a proximal direction when the first elongate inner body is advanced distally with respect to the arm. The manipulator can further include a second elongate inner body having a proximal end and a distal end that is slidably disposed within the at least one elongate passage of the elongate tubular arm that can be slidably disposed with respect to the first inner body. The distal end of the second inner elongate body can be biased to curl away from the longitudinal axis toward the deployed proximally oriented distal end of the first elongate inner body when the second elongate inner body is advanced distally with respect to the arm.

At least one of the elongate tubular arm, first elongate inner body or second elongate inner body can be connected to an axial actuator, wherein the actuator is configured to advance the component to which it is connected along a direction parallel to the longitudinal axis. Moreover, at least one of the elongate tubular arm first elongate inner body or second elongate inner body can be connected to a rotational actuator, wherein the rotational actuator is configured to rotate one or more of the elongate tubular arm, first elongate inner body and second elongate inner body.

At least one of the first elongate inner body and second elongate inner body can include an end effector attached thereto configured to perform at least one of a cutting, grasping, irrigating, evacuating, viewing or suctioning function. If desired, the end effector can include one or more of an electrosurgical device, a blade, and an ultrasonic transducer.

The disclosure also provides implementations of a laparoscopic, urinary, gynecological, neurological, or orthopedic surgical procedure utilizing the catheters or robotic manipulators disclosed herein. The disclosed catheters/manipulators can also be used in any suitable minimally invasive procedure, or a percutaneous procedure.

For example, the percutaneous procedure can include utilizing one or more of the disclosed devices to access a patient's sinus passages. The devices can be used, for example, to remove one or more polyps, and can even be used to breach a thin bony layer within the sinuses to access the cranial cavity to perform a procedure inside the cranial cavity.

In other embodiments, the percutaneous procedures disclosed herein can include an ablation procedure, such as within the heart of a patient or elsewhere, as well as a cryoablation procedure. The disclosure also provides suitable handles and actuators (illustrated in the Appendix of U.S. Provisional Application Serial No. 62/567,203, filed October 2, 2017, for example) for controlling one or more of the first elongate inner body, second elongate inner body and elongate tubular main body.

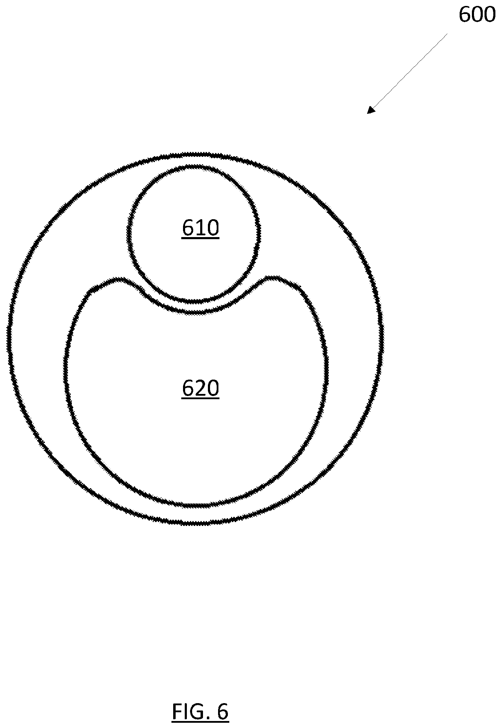

In further accordance with the present disclosure, Fig. 6 illustrates a cross sectional view of an extruded main body portion 600 of a further embodiment of a catheter. The body includes an extrusion defining two offset channels 610, 620. A first channel 610 is illustrated as having a generally circular cross-section, and the second channel 620 that is parallel thereto is illustrated as having a cross-section that is circular with a scalloped portion removed in order to accommodate the channel with the circular cross section. The main body 600 can be made from any suitable polymeric material, such as those set forth herein. The main body can be formed from a multilayer polymeric extrusion with one or more reinforcement (e.g. layers of braiding) formed thereon or therein. The main body can be coated with any suitable coating or material to enhance its lubricity, as desired.

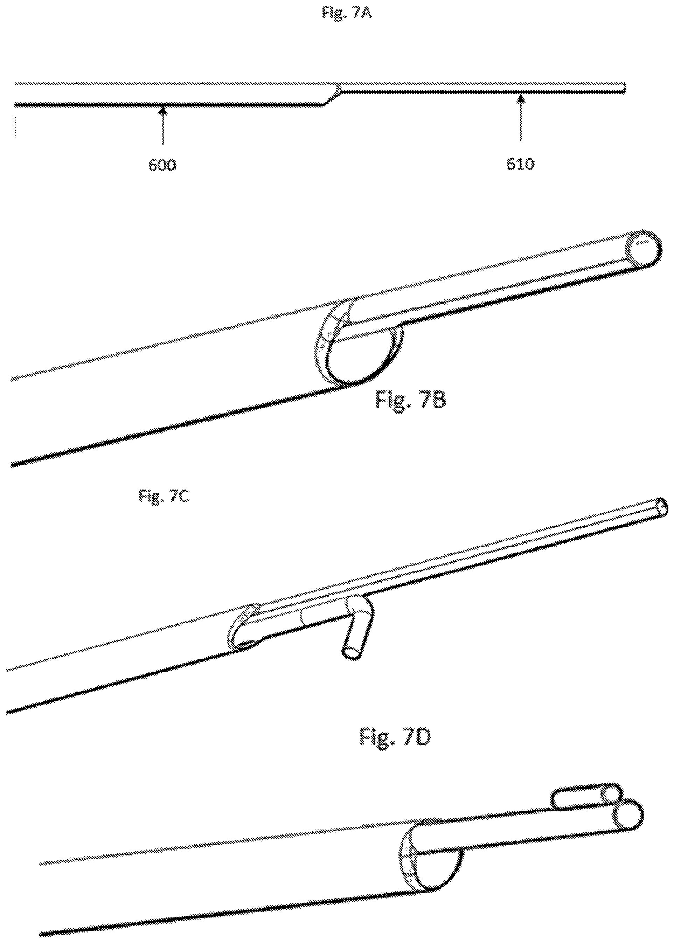

Fig. 7A presents a side view of the illustrative catheter of Fig. 6 including the main shaft 600 described above, provided with at least one braided layer. The catheter further includes a distal tubular segment extending distally from main shaft 600 that defines therein lumen 610 that is radially co-located with the first channel of the main body. For example, the distal tubular segment can be an extruded tube that extends the full length along the inside of the main body to a proximal end of the catheter. The distal tubular segment may similarly be braided if desired, may be pre-curved as described elsewhere herein and/or can be deflectable, for example, by providing a pull wire within the lumen of the distal segment, or within a co-extruded lumen of the distal segment (not specifically illustrated). A distal end of the pull wire (not shown) can be attached to a collar embedded within or on the distal tubular segment, as desired. As illustrated in Fig. 7B, the distal tubular segment and its associated channel that it surrounds can be used to act as a guidewire lumen, permitting the catheter to be used as an over the wire catheter, or for delivering a lower profile catheter therethrough, such as a snare catheter, as set forth in further detail below.

Fig. 7C illustrates an embodiment wherein the larger/major, e.g., non-circular, lumen defined in the main body can act as a delivery lumen for a catheter that can be steerable (e.g., by a steering wire) or that can have a curve preformed into it (e.g., by heating and bending the catheter if polymeric in composition) that the catheter can assume after it is advanced distally out of the distal end of the major lumen of the main body. As presented in Fig. 7D, the distal tubular segment can be provided with a further tubular member disposed thereon, or integrated therewith in a co-extrusion, that can act as a guidewire lumen to facilitate a rapid exchange ("RX") procedure with the guidewire rather than having the guidewire traverse the entire length of the catheter as in an over the wire ("OTW") procedure.

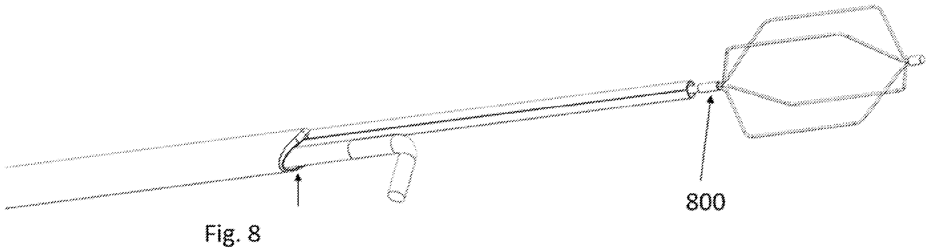

Fig. 8 presents the embodiment of Figs. 7A-7D, but including a snare catheter 800 disposed through the minor lumen for effectuating capture, for example, of a guidewire in a mitral cerclage procedure as set forth in U.S. Patent Application Serial No. 15/796,344, filed October 27, 2017. Further aspects of the snare catheter can be seen in that application, as well as in U.S. Provisional Patent Application Serial No. 62/615,309, filed January 9, 2018. The present catheter can be used, for example, for such mitral cerclage procedures. For example, the snare catheter can be used to capture a guidewire while the major passage accommodates an articulating catheter as described hereinabove for grasping a cardiac valve leaflet, or other structure.

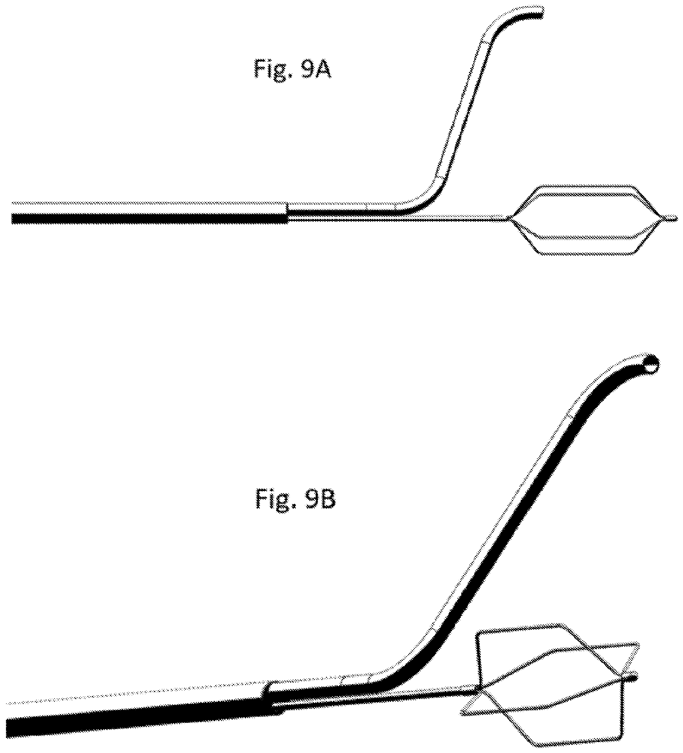

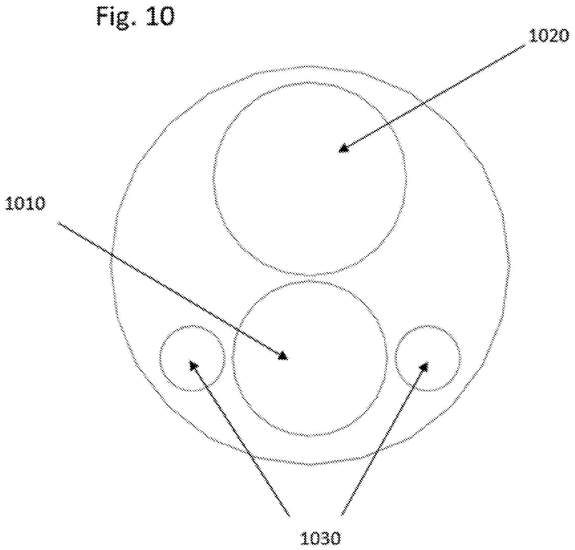

A further embodiment is presented in Figs. 9A-9B, which illustrates an articulating catheter having two preformed bends that resume their bent shape when advanced distally from the main catheter. Fig. 10 illustrates a further possible cross section for the main catheter, wherein major and minor lumens 1010, 1020 are presented, but two additional steering wire lumens 1030 are presented. If desired, further steering wire lumens are presented that can be used for housing a pull wire that is attached at its distal end to a portion of the catheter (not shown), such as to a ring collar that is formed on or in the body of the catheter.

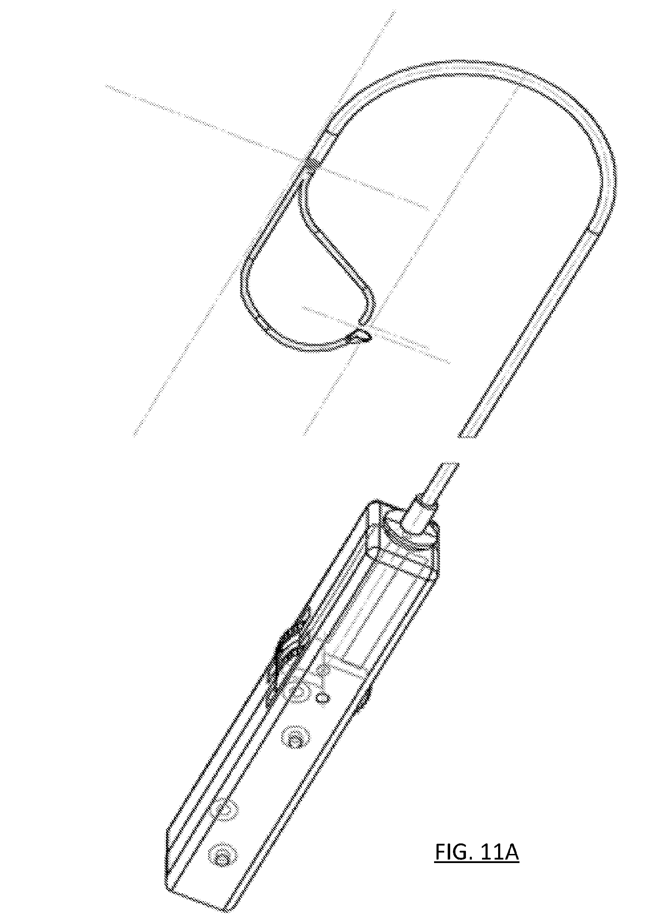

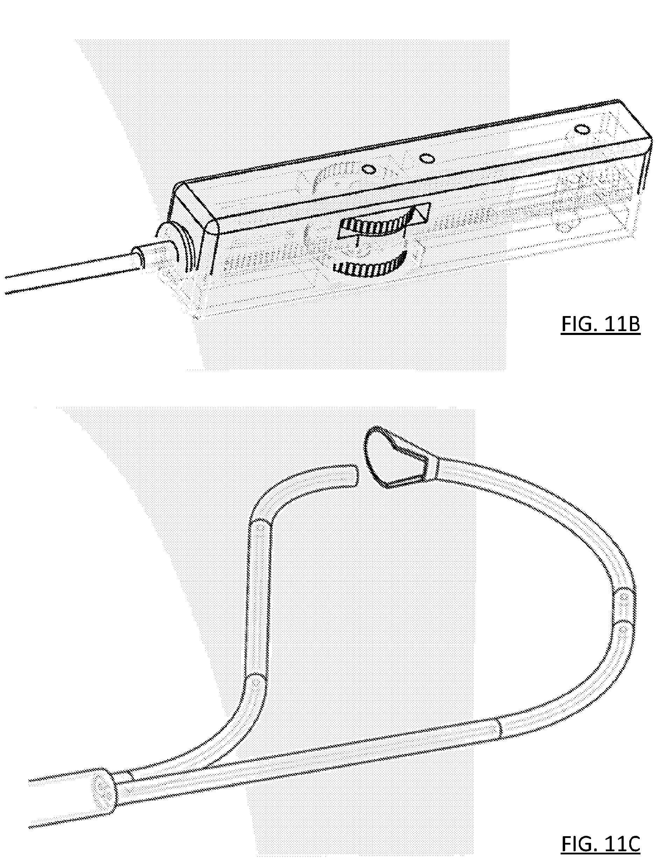

Figs. 11A-11C display a further embodiment of a catheter in accordance with the disclosure (or aspects thereof) that includes a scoop on one of the articulating arms as presented. The scoop, or funnel, can help guide the other articulating arm into contact with it. If desired, permanent magnets can be added at the end of each articulating arm (not shown), or a winding around each end of the catheter can be made to form a solenoid on the end of each of the arms (not shown). When electrical current is run along the same helical direction through each solenoid, the created magnetic fields add to each other, and attract each other, causing the arms to move more closely together into contact. The force is directly proportional to the current that passes through the windings. Also illustrated is a push-pull actuator for relatively articulating each of the deployable limbs in the catheter. The disclosed catheter uses a toothed wheel, or gear, that rotates around an axle and engages a gear rack in a sliding track that in turn is attached to one of the articulating arms.

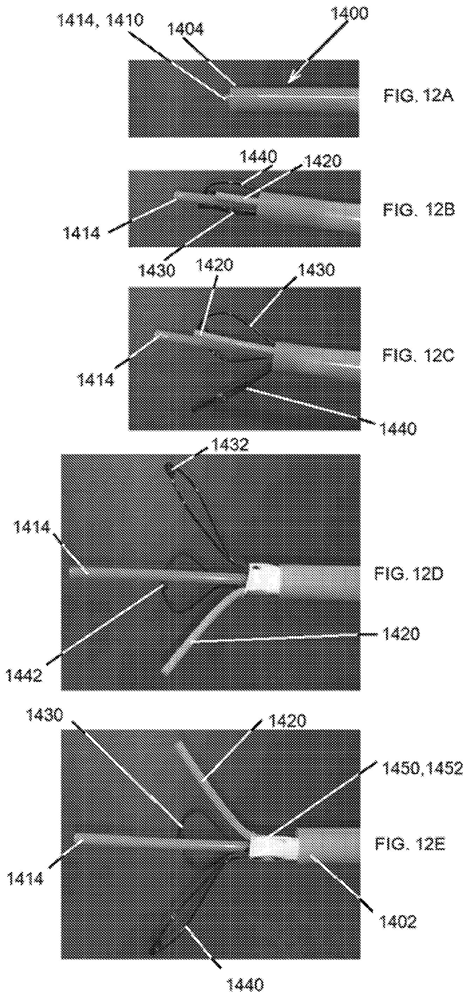

For purposes of illustration, and not limitation, FIGS. 12A-13C depict yet another embodiment of a catheter in accordance with the present disclosure.

FIG. 12 illustrates a further embodiment 1400 of a catheter. The distal end 1404 of catheter 1400 is depicted to highlight its functionality. Catheter 1400 also includes a proximal end and elongate body (not shown) having one or more actuators to manipulate the various sub-components of catheter 1400 described in detail below. Catheter 1400 is defined by an outer tubular member having a proximal end, a distal end 1404, and defines an elongate passage therethrough along its length. Elongate passage slidably accommodates an intermediate tubular member 1450 therein having a proximal end (not shown), a distal end 1452 and in turn also defining a passage along its length for slidably receiving a subassembly therein including at least one further catheter, tool or manipulator. As illustrated in FIGS. 12A-E, a subassembly is provided slidably received within intermediate tubular member 1450 that includes a central tubular member 1410 having a proximal end, a distal end 1414, and defining a passage along its length, for example, for receiving a guidewire for guiding catheter 1400 to a target location. As illustrated, central tubular member 1410 is a straight member, but can be imparted with a curvature if desired. The subassembly further includes a second tubular member 1420 having a proximal end (not shown), a distal end 1424 and an elongate body defining a central lumen along its length. Second tubular member 1420, as illustrated, has a curvature imparted to it. Also provided are collapsible loops 1430, 1440, which may be made from any suitable material. The particular loops illustrated are formed from nitinol. Each loop is defined by a filament that can include a stress distribution loop (1432, 1442) formed therein that traverses 360 degrees or more. Providing a stress distribution loop facilitates collapse of the loops 1430, 1440 by distributing the bending stress over a longer effective length of wire. The material from which loops 1430, 1440 is formed can extend to the proximal end of the catheter 1400, or may be secured in the distal ends of additional tubular members (not shown) that are slidably disposed in intermediate tubular member 1450. Loops can be made, for example, from shape memory material such as various nickel titanium alloys.

As illustrated, the subassembly within tubular member 1450 can be both slidably and rotatably movable with respect to the outer tubular member of catheter 1400. If desired, each of the subcomponents 1410, 1420, 1430 and 1440 may be slidably and rotatably movable with respect to each other, and the main body of the catheter 1400 as well as the intermediate tubular member 1450.

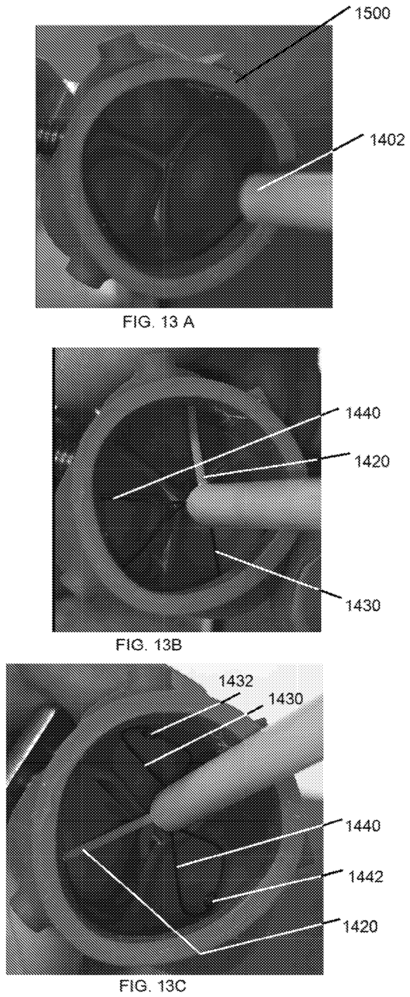

As illustrated in FIGS. 13A-13C, the embodiment 1400 is illustrated in use with respect to the structure of a tricuspid valve. In use, after the distal end 1402 of catheter 1400 is advanced, for example, to a tricuspid valve, the subassembly housed within intermediate tubular member 1450 is advanced distally out of distal end 1402 of catheter 1400, and the distal end 1414 of central tubular member 1410 can be directed through the center of the tricuspid valve between the leaflets. Next, the two loops 1430, 1440 are deployed and advanced under the leaflet against the center of each leaflet by the valve annulus. This permits tubular member 1420 to be positioned at the center of the third leaflet by the annulus. At this time, any desired instrument, such as a cutting wire or piercing instrument can be advanced through the leaflet at its edge by the annulus, such as to advance a electrosurgical cutting wire through the leaflet, permitting the cutting wire to be dragged radially inwardly through the leaflet to cut the leaflet in half. In accordance with a further example, a suture can be anchored by subassembly component 1420. The suture can then be used as a guide rail for delivering a prosthesis to be implanted over the leaflet, with our without cutting it in half first. It will be appreciated that catheter 1400 can be used in many different types of procedures and that these illustrations are only examples.

The devices and methods disclosed herein can be used for other procedures in an as-is condition, or can be modified as needed to suit the particular procedure. In view of the many possible embodiments to which the principles of this disclosure may be applied, it should be recognized that the illustrated embodiments are only preferred examples of the disclosure and should not be taken as limiting the scope of the invention, which is defined by the claims.

## Claims

1. An elongate catheter having a proximal end and a distal end comprising:
a) an elongate tubular main body (22) having a proximal end, a distal end, and defining at least one elongate passage therethrough, the elongate tubular main body defining a longitudinal axis along its length;
b) a first elongate inner body (10) having a proximal end and a distal end that is slidably disposed within the at least one elongate passage of the elongate tubular main body (22), the distal end of the first inner elongate body being configured to curl away from the longitudinal axis in a proximal direction when the first elongate inner body (10) is advanced distally with respect to the main body (22); and
c) a second elongate inner body (20) having a proximal end and a distal end that is slidably disposed within the at least one elongate passage of the elongate tubular main body (22), and slidably disposed with respect to the first inner body (10), the distal end of the second inner elongate body (20) being configured to curl away from the longitudinal axis toward the deployed proximally oriented distal end of the first elongate inner body (10) when the second elongate inner body is advanced distally with respect to the main body (22),
wherein:
the first elongate inner body (10) and second elongate inner body (20) each define a lumen along their respective lengths;
the lumen of the second elongate inner body (20) includes a movable body slidably disposed therein having a distal end, the movable body including a guidewire (300) having an electrically conductive core wire (320) surrounded by a jacket (310) made from dielectric material, wherein the jacket is removed from a portion of the core wire along one side of the core wire to create an exposed region (330) of the core wire; and
the distal end of the movable body is configured to penetrate a target tissue region prior to passing into the lumen of the first elongate inner body (10).

2. The elongate catheter of Claim 1, wherein the lumen of the first elongate inner body (10) includes a snare catheter configured to be deployed from the distal end of the first elongate inner body to capture the guidewire (300) when the guidewire is deployed from the distal end of the second elongate inner body (20).

3. The elongate catheter of Claim 1, wherein the core wire (406) is surrounded by a first insulating layer (408), the first insulating layer is surrounded by a second electrical conductor (410), and the second electrical conductor is surrounded by an outer insulating layer (420), wherein the outer insulating layer is removed to expose a portion of the second electrical conductor to define an exposed portion (424) of the second electrical conductor.

4. The elongate catheter of Claim 3, wherein the second electrical conductor (410) is formed from an electrically conductive tube.

5. The elongate catheter of Claim 4, wherein the electrically conductive tube is defined by a tubular body that in turn defines at least one opening therethrough.

6. The elongate catheter of Claim 4, wherein the at least one opening is spiral shaped and winds around the first insulating layer (408).

7. The elongate catheter of Claim 4, wherein the at least one opening and the tubular body define a plurality of articulating segments.

8. The elongate catheter of Claim 1, wherein the movable body further includes a dart passer that is configured to advance a dart having a suture attached thereto out of the distal end of the second elongate inner body (20) and into a receiving cuff disposed in the lumen of the first elongate inner body (10).

9. The elongate catheter of Claim 8, wherein the receiving cuff is attached to a filament that passes through the lumen of the first elongate inner body (10).

10. The elongate catheter of Claim 1, wherein the rotational position of the first elongate inner body (10) is fixed with respect to the rotational position of the second elongate inner body (20).

11. The elongate catheter of Claim 1, wherein the core wire (320) is bent in half so that the exposed region (330) of the core wire faces itself.

12. A system including the elongate catheter of Claim 11, wherein the core wire (320) includes electrically conductive exposed first and second ends (340) coupled to an electrosurgical generator to cause current to pass through the core wire to cause an arc discharge to develop in the exposed region (330) of the core wire that jumps across a gap between facing exposed surfaces of the core wire in the exposed region of the core wire that can help cut and/or burn through tissue by pulling the exposed wire through the tissue.

13. An electrosurgical system including a radio frequency power supply operably coupled to first and second electrically conductive ends (340) of the electrically conductive core wire (320) of the elongate catheter of any of claims 1 to 11.

## Patentansprüche

1. Länglicher Katheter, der ein proximales Ende und ein distales Ende aufweist, umfassend:
a) einen länglichen rohrförmigen Hauptkörper (22), der ein proximales Ende, ein distales Ende aufweist und mindestens einen länglichen Durchlass dadurch definiert, wobei der längliche rohrförmige Hauptkörper eine Längsachse entlang seiner Länge definiert;
b) einen ersten länglichen Innenkörper (10), der ein proximales Ende und ein distales Ende aufweist und der verschiebbar innerhalb des mindestens einen länglichen Durchlasses des länglichen rohrförmigen Hauptkörpers (22) angeordnet ist, wobei das distale Ende des ersten inneren länglichen Körpers dazu konfiguriert ist, sich von der Längsachse weg in einer proximalen Richtung zu krümmen, wenn der erste längliche Innenkörper (10) in Bezug auf den Hauptkörper (22) distal vorgeschoben wird; und
c) einen zweiten länglichen Innenkörper (20), der ein proximales Ende und ein distales Ende aufweist und der verschiebbar innerhalb des mindestens einen länglichen Durchlasses des länglichen rohrförmigen Hauptkörpers (22) angeordnet ist und verschiebbar in Bezug auf den ersten Innenkörper (10) angeordnet ist, wobei das distale Ende des zweiten inneren länglichen Körpers (20) dazu konfiguriert ist, sich von der Längsachse weg in Richtung des entfalteten proximal ausgerichteten distalen Endes des ersten länglichen Innenkörpers (10) zu krümmen, wenn der zweite längliche Innenkörper in Bezug auf den Hauptkörper (22) distal vorgeschoben wird,
wobei:
der erste längliche Innenkörper (10) und der zweite längliche Innenkörper (20) jeweils ein Lumen entlang ihrer jeweiligen Länge definieren;
das Lumen des zweiten länglichen Innenkörpers (20) einen bewegbaren Körper beinhaltet, der verschiebbar darin angeordnet ist und ein distales Ende aufweist, wobei der bewegbare Körper einen Führungsdraht (300) beinhaltet, der einen elektrisch leitenden Kerndraht (320) aufweist, der durch einen Mantel (310) aus dielektrischem Material umgeben ist, wobei der Mantel von einem Abschnitt des Kerndrahts entlang einer Seite des Kerndrahts entfernt wird, um eine freiliegende Region (330) des Kerndrahts zu erzeugen; und
das distale Ende des bewegbaren Körpers dazu konfiguriert ist, eine Zielgeweberegion zu durchdringen, bevor es in das Lumen des ersten länglichen Innenkörpers (10) gelangt.

2. Länglicher Katheter nach Anspruch 1, wobei das Lumen des ersten länglichen Innenkörpers (10) einen Schlingenkatheter beinhaltet, der dazu konfiguriert ist, von dem distalen Ende des ersten länglichen Innenkörpers entfaltet zu werden, um den Führungsdraht (300) einzufangen, wenn der Führungsdraht von dem distalen Ende des zweiten länglichen Innenkörpers (20) entfaltet wird.

3. Länglicher Katheter nach Anspruch 1, wobei der Kerndraht (406) durch eine erste Isolierschicht (408) umgeben ist, die erste Isolierschicht durch einen zweiten elektrischen Leiter (410) umgeben ist und der zweite elektrische Leiter durch eine äußere Isolierschicht (420) umgeben ist, wobei die äußere Isolierschicht entfernt wird, um einen Abschnitt des zweiten elektrischen Leiters freizulegen, um einen freiliegenden Abschnitt (424) des zweiten elektrischen Leiters zu definieren.

4. Länglicher Katheter nach Anspruch 3, wobei der zweite elektrische Leiter (410) aus einem elektrisch leitfähigen Rohr gebildet ist.

5. Länglicher Katheter nach Anspruch 4, wobei das elektrisch leitfähige Rohr durch einen rohrförmigen Körper definiert ist, der wiederum mindestens eine Öffnung dadurch definiert.

6. Länglicher Katheter nach Anspruch 4, wobei die mindestens eine Öffnung spiralförmig ist und um die erste Isolierschicht (408) gewunden ist.

7. Länglicher Katheter nach Anspruch 4, wobei die mindestens eine Öffnung und der rohrförmige Körper eine Vielzahl von gelenkigen Segmenten definieren.

8. Länglicher Katheter nach Anspruch 1, wobei der bewegbare Körper ferner eine Pfeilführeinrichtung beinhaltet, die dazu konfiguriert ist, einen Pfeil mit einem daran angebrachten Faden aus dem distalen Ende des zweiten länglichen Innenkörpers (20) und in eine in dem Lumen des ersten länglichen Innenkörpers (10) angeordnete Aufnahmemanschette vorzuschieben.

9. Länglicher Katheter nach Anspruch 8, wobei die Aufnahmemanschette an einem Filament angebracht ist, das durch das Lumen des ersten länglichen Innenkörpers (10) verläuft.

10. Länglicher Katheter nach Anspruch 1, wobei die Drehposition des ersten länglichen Innenkörpers (10) in Bezug auf die Drehposition des zweiten länglichen Innenkörpers (20) fixiert ist.

11. Länglicher Katheter nach Anspruch 1, wobei der Kerndraht (320) in zwei Hälften gebogen ist, sodass die freiliegende Region (330) des Kerndrahts sich selbst zugewandt ist.

12. System, beinhaltend den länglichen Katheter nach Anspruch 11, wobei der Kerndraht (320) ein elektrisch leitendes freiliegendes erstes und zweites Ende (340) beinhaltet, die an einen elektrochirurgischen Generator gekoppelt sind, um zu bewirken, dass Strom durch den Kerndraht fließt, um zu bewirken, dass sich eine Bogenentladung in der freiliegenden Region (330) des Kerndrahts entwickelt, die über einen Spalt zwischen gegenüberliegenden freiliegenden Oberflächen des Kerndrahts in der freiliegenden Region des Kerndrahts springt, und die zum Schneiden und/oder Durchbrennen von Gewebe beitragen kann, indem der freiliegende Draht durch das Gewebe gezogen wird.

13. Elektrochirurgisches System, beinhaltend eine Funkfrequenzleistungsversorgung, die betriebsfähig an ein erstes und zweites elektrisch leitfähigen Ende (340) des elektrisch leitfähigen Kerndrahts (320) des länglichen Katheters nach einem der Ansprüche 1 bis 11 gekoppelt ist.

## Revendications

1. Cathéter allongé possédant une extrémité proximale et une extrémité distale comprenant :
a) un corps principal tubulaire allongé (22) possédant une extrémité proximale, une extrémité distale et définissant au moins un passage allongé à travers celles-ci, le corps principal tubulaire allongé définissant un axe longitudinal sur sa longueur ;
b) un premier corps interne allongé (10) possédant une extrémité proximale et une extrémité distale qui est disposée de manière coulissante à l'intérieur dudit au moins un passage allongé du corps principal tubulaire allongé (22), l'extrémité distale du premier corps allongé interne étant conçue pour s'enrouler en s'éloignant de l'axe longitudinal suivant une direction proximale lorsque le premier corps interne allongé (10) est avancé de manière distale par rapport au corps principal (22) ; et
c) un second corps interne allongé (20) possédant une extrémité proximale et une extrémité distale qui est disposée de manière coulissante à l'intérieur dudit au moins un passage allongé du corps principal tubulaire allongé (22), et disposée de manière coulissante par rapport au premier corps interne (10), l'extrémité distale du second corps allongé interne (20) étant conçue pour s'enrouler en s'éloignant de l'axe longitudinal en direction de l'extrémité distale déployée orientée de manière proximale du premier corps interne allongé (10) lorsque le second corps interne allongé est avancé de manière distale par rapport au corps principal (22),
ledit premier corps interne allongé (10) et ledit second corps interne allongé (20) définissant chacun une lumière le long de leurs longueurs respectives ;
ladite lumière du second corps interne allongé (20) comprenant un corps mobile disposé de manière coulissante en son sein et possédant une extrémité distale, ledit corps mobile comprenant un fil de guidage (300) possédant un fil d'âme électriquement conducteur (320) entouré d'une gaine (310) fabriquée à partir d'un matériau diélectrique, ladite gaine étant retirée d'une partie du fil d'âme le long d'un côté du fil d'âme de manière à créer une zone exposée (330) du fil d'âme ; et
ladite extrémité distale du corps mobile étant conçue pour pénétrer dans une zone tissulaire cible avant de passer dans la lumière du premier corps interne allongé (10).

2. Cathéter allongé selon la revendication 1, ladite lumière du premier corps interne allongé (10) comprenant un cathéter de reprise conçu pour être déployé à partir de l'extrémité distale du premier corps interne allongé de manière à capturer le fil de guidage (300) lorsque le fil de guidage est déployé à partir de l'extrémité distale du second corps interne allongé (20).

3. Cathéter allongé selon la revendication 1, ledit fil d'âme (406) étant entouré par une première couche isolante (408), ladite première couche isolante étant entourée par un second conducteur électrique (410), et ledit second conducteur électrique étant entouré par une couche isolante externe (420), ladite couche isolante externe étant retirée de manière à exposer une partie du second conducteur électrique afin de définir une partie exposée (424) du second conducteur électrique.

4. Cathéter allongé selon la revendication 3, ledit second conducteur électrique (410) étant formé à partir d'un tube électriquement conducteur.

5. Cathéter allongé de la revendication 4, ledit tube électriquement conducteur étant défini par un corps tubulaire qui à son tour définit au moins une ouverture à travers lui.

6. Cathéter allongé selon la revendication 4, ladite au moins une ouverture étant en forme de spirale et s'enroulant autour de la première couche isolante (408).

7. Cathéter allongé selon la revendication 4, ladite au moins une ouverture et ledit corps tubulaire définissant une pluralité de segments d'articulation.

8. Cathéter allongé selon la revendication 1, ledit corps mobile comprenant en outre un dispositif de passage de fléchette qui est conçu pour faire avancer une fléchette à laquelle est fixée une suture hors de l'extrémité distale du second corps interne allongé (20) et dans un manchon de réception disposé dans la lumière du premier corps interne allongé (10).

9. Cathéter allongé selon la revendication 8, ledit manchon de réception étant fixé à un filament qui passe à travers la lumière du premier corps interne allongé (10).

10. Cathéter allongé selon la revendication 1, ladite position de rotation du premier corps interne allongé (10) étant fixe par rapport à la position de rotation du second corps interne allongé (20).

11. Cathéter allongé selon la revendication 1, ledit fil d'âme (320) étant plié en deux de sorte que la zone exposée (330) du fil d'âme se fasse face à elle-même.

12. Système comprenant le cathéter allongé selon la revendication 11, ledit fil d'âme (320) comprenant des première et seconde extrémités exposées électriquement conductrices (340) couplées à un générateur électrochirurgical de manière à amener le courant à passer à travers le fil d'âme afin de provoquer le développement d'une décharge en arc dans la zone exposée (330) du fil d'âme qui saute à travers un espace entre les surfaces exposées en regard du fil d'âme dans la zone exposée du fil d'âme qui peut aider à découper et/ou brûler à travers le tissu en tirant le fil exposé à travers le tissu.

13. Système électrochirurgical comprenant une alimentation électrique à radiofréquence couplée de manière fonctionnelle aux première et seconde extrémités électriquement conductrices (340) du fil d'âme électriquement conducteur (320) du cathéter allongé selon l'une quelconque des revendications 1 à 11.
